Europäisches Patentamt

European Patent Office

Office européen des brevets

(11) Publication number: **0 082 950**
**B1**

(12) **EUROPEAN PATENT SPECIFICATION**

(45) Date of publication of patent specification: **02.04.86**

(51) Int. Cl.⁴: **A 61 B 1/12,** A 61 B 1/00, F 16 L 37/00

(21) Application number: **82110607.7**

(22) Date of filing: **17.11.82**

(54) **Liquid supply device for an endoscope.**

(30) Priority: **19.11.81 JP 185759/81**

(43) Date of publication of application:
**06.07.83 Bulletin 83/27**

(45) Publication of the grant of the patent:
**02.04.86 Bulletin 86/14**

(84) Designated Contracting States:
**AT BE CH DE FR GB IT LI NL SE**

(56) References cited:
**EP-A-0 044 018**
**DE-A-2 303 147**
**DE-A-2 948 564**
**DE-B-1 228 471**
**FR-A-2 312 719**
**FR-A-2 423 437**
**US-A-3 903 877**

(73) Proprietor: **OLYMPUS OPTICAL CO., LTD.**
**43-2, 2-chome, Hatagaya Shibuya-ku**
**Tokyo 151 (JP)**

(72) Inventor: **Ohshima, Yutaka**
**1-60-7, Sumiyoshi-cho**
**Fuchu-shi Tokyo (JP)**

(74) Representative: **KUHNEN & WACKER**
**Patentanwaltsbüro**
**Schneggstrasse 3-5 Postfach 1729**
**D-8050 Freising (DE)**

Courier Press, Leamington Spa, England.

## Description

This invention relates to a liquid supply device for an endoscope, which is adapted to supply a liquid such as water or a liquid medicine to a liquid supply passage of the endoscope.

An endoscope generally has a liquid supply passage therein and a liquid inlet provided in a connector connected to, for example, a forward end of a universal cord and a liquid outlet provided at a distal end, for example an insertion section, of the endoscope. For example, a liquid medicine is supplied through the liquid supply passage into, for example, a body cavity of a human being to permit it to be applied to a region of interest, or cleaning water is supplied through the liquid passage to permit the surface of an objective lands to be cleaned. As a liquid supply device of this type adapted to supply a liquid such as water or a liquid medicine, from US—A—3,903,877 a device is known in which the ends of an air supply tube and a liquid supply tube are connected to a liquid supply tank, the other end of the liquid supply tube is removably connected to a liquid inlet of a connector through a connecting cap and in this configuration compressed air is supplied from the air supply pump through the air supply tube into the liquid supply tank, causing internal pressure in the liquid supply tank to be increased to permit the liquid to be supplied into a liquid supply passage of an endoscope through the liquid supply tube.

When in this device the connecting cap for the liquid supply tube is removed from the connector of the endoscope upon completion of a liquid supply operation, the liquid in the liquid supply tank leaks out through the liquid supply tank, causing disadvantages such as the contamination of an endoscope inspection chamber, etc.

It is therefore the object of this invention to provide a liquid supply device adapted to prevent a liquid in a liquid supply tank from flowing out through a liquid supply tube, when said liquid supply tube is removed from the endoscope upon completion of a liquid supply operation.

Solution of this object is achieved by the features of claim 1 or claim 5.

This invention can be more fully understood from the following detailed description when taken in conjunction with the accompanying drawings, in which:

Fig. 1 is a perspective view generally showing a liquid supply device connected to an endoscope;

Fig. 2 is a schematic view generally showing the liquid supply device according to one embodiment of this invention;

Fig. 3 is a cross-sectional view showing a state in which a part of the liquid supply device is opposed to a connecting section of the endoscope;

Fig. 4 is a cross-sectional view taken along line 4—4 in Fig. 3, showing a part of the liquid supply device;

Fig. 5 is a view, similar to that of Fig. 3, showing a state in which the liquid supply device is connected to the connecting section;

Fig. 6 is a cross-sectional view showing a liquid supply device according to another embodiment of this invention; and

Fig. 7 is a cross-sectional view showing a state in which the liquid supply device is connected to the connecting section of the endoscope.

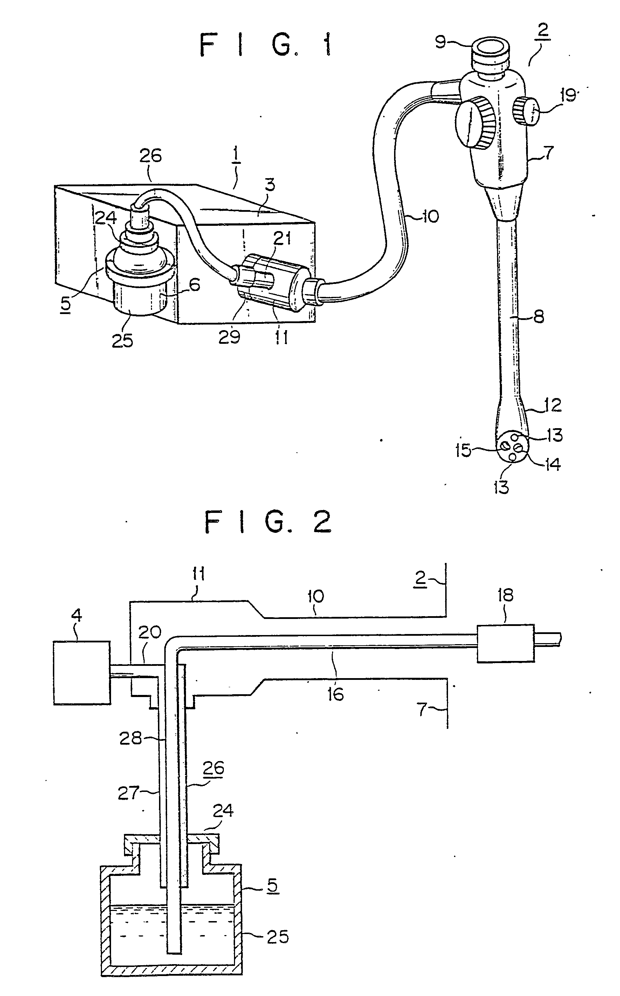

The embodiments of this invention will be explained below by referring to Figs. 1 to 5.

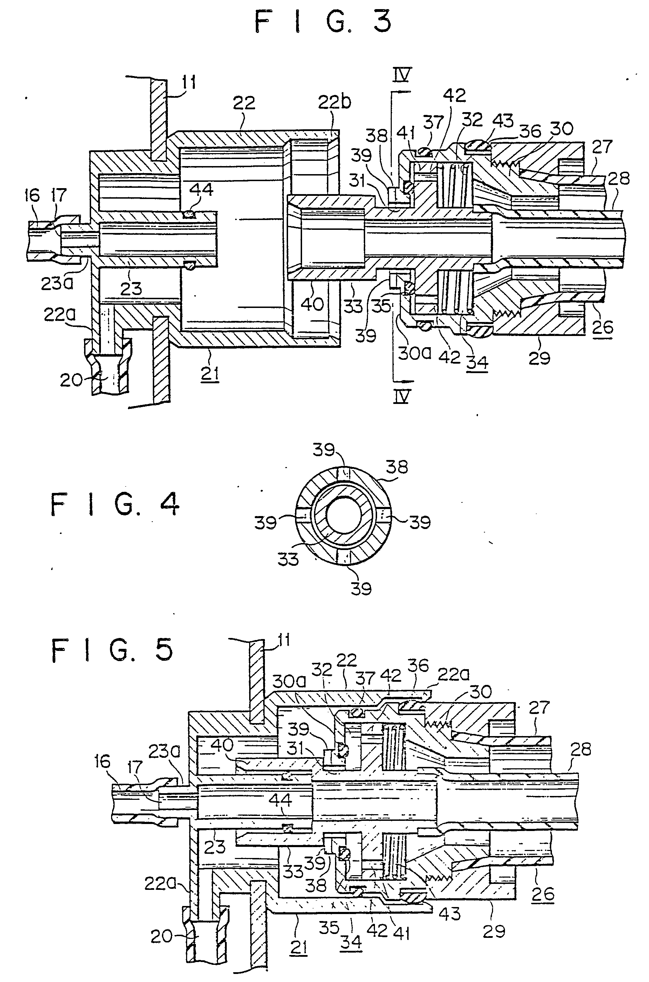

Fig. 1 shows the state in which an endoscope 2 is connected to a liquid supply device body 1. The liquid supply device body 1 is formed integrally with, for example, a light source, and an air supply pump 4 (Fig. 2) and a light source lamp (not shown) and so on are mounted within a case 3. A liquid supply tank 5 is detachably mounted by an attaching ring 6 on the outer side surface of the case 3. The endoscope 2 includes a control section 7 and an insertion section 8. An eyepiece section 9 is provided on the control section 7 to which a universal cord 10 is connected. A connector 11 is disposed on one end of the universal code 10 such that it is detachably connected to the liquid supply device 1. A distal end structure 12 is provided at the forward end of the insertion section 8. At the distal end structure an illumination window 13 connected to an illumination optical system, an object lens 14 connected to an observation optical system, and a liquid outlet 15 are provided. As shown in Fig. 2, a liquid passage 16 is formed in the endoscope 2 and extends through the universal cord 10, operation section 7 and insertion section 8. The liquid passage 16 communicates at one end with a liquid inlet of the connector and at the other end with the liquid outlet 15. An openable valve 18 is disposed in the liquid passage of the control section 7 and is adapted to be externally operated by an operation button 19 (Fig. 1) on the control section 7 such that it opens and closes the liquid passage. The ends of the liquid passage 16 and air supply passage 20 are formed in the connector 11. A connecting member 21, Fig. 3, is projected on the outer side portion of the connector 11 and comprises a first, large-diameter annular member 22 and a second, small-diameter annular member 23 coaxial with the first annular member 22 with an annular gap defined therein, thereby providing a double-tube structure. A connecting port 22a is projected on that outer peripheral surface of the first annular member 22 which is located near the base end of the first annular member. One end of the air passage 20 is connected to the connecting port 22a and the second annular member 23 is connected to a liquid guide inlet 17 of the liquid passage 16 through a connecting port 23a which is provided on the base end of the second annular member 23. The liquid supply tank 5 has its upper open end covered by a cover 24 and is comprised of a sealed container 25 for holding water, a liquid medicine, etc., therein. The cover 24 is attached to the base end portion of a connecting tube 26. The connecting tube 26 is comprised of a large-diameter air supply tube 27 and a small-diameter liquid supply tube 28 within the large-diameter air supply tube 27, providing a double-tube structure. The basic ends of the air supply tube 27 and liquid supply tube 28 extend through the cover 24 into the sealed container 25 such that, as shown in Fig. 2, the open end of the air supply pipe 27 is located in a higher position than that of the open end of the liquid supply tube 28. A cylindrical cap 29 is fitted over the forward end portion of the connecting tube 26. An air supply cap 30 is threadably inserted into the cylindrical cap 29 as shown in Fig. 3 and can be inserted into the first annular member 22 of the connecting member 21. The cap 30 takes the form of a bottomed cylinder and has its open end secured to the other end of the air supply tube 27. A circular opening 31 is formed in a base 30a of the air supply cap 30 and a cylinder section 32 is formed in communication with the opening 31 to provide a closable valve mechanism 34 together with a liquid supply cap 33 as will be described later. An O-ring 35 is located in an inner, recessed portion of the bottom 30a of the cap 30 and C- and an O-rings (36 and 37) are fitted in the annular recessed portions of the outer periphery of the cap 30. Stated in more detail, the C-ring 36 is forced into the annular-recessed portion of the outer periphery of the cap 30 and an outer periphery part of the C-ring 36 is somewhat projected from the outer periphery of the cap 30. A ring like projection 38 is formed on the outer portion of the bottom 30a of the cap 30 such that it is coaxial with the circular opening 31, and has a plurality of through holes 39 (4 in this embodiment) as shown in Fig. 4 which extend at predetermined intervals from the inner surface of the cap 30 toward the outer periphery of the cap 30. The liquid supply cap 33 is cylindrical in configuration and inserted into the opening 31 of the cap 30 with a predetermined gap therebetween so that it can be moved back and forth relative to the cap 30. The cap 33 has a connecting section 40 which is projected forward of the cap 30 and adapted to receive the second annular member 23 of the connecting member 21. The other end of the liquid supply tube 28 is fitted over the base end of the cap 33. An outwardly extending flange-like piston section 41 is disposed at the intermediate portion of the cap 33 and adapted to be moved in the cylinder section 32 of the cap 30 such that it can be moved back and forth. The interior of the cylinder section 32 on the side of the air supply tube 27 communicates with the interior of the cylinder section 32 on the side of the bottom 30a through a plurality of communication holes 42 extending through the piston section 41. A compression coil spring 43 is held within the portion of the cylinder section 32 which is located on the side of the air supply tube 27, and adapted to forwardly urge the piston section 41. In the nonuse state in which the connecting portion 40 of the cap 33 is not engaged with the second annular member 23, the cap 33 is held, by the compression coil spring 43, urged against the inner surface of the bottom 30a of the cap 30. Reference numeral 44 shows an O-ring fitted in the recessed portion of the outer periphery of the second annular member 23 in the connecting member 21.

The operation of a liquid supply device so constructed will be explained now in detail:

As shown in Fig. 1, the connector 11 of the endoscope 1 is connected to the liquid supply device body 1 and the cap 29 for the connecting tube 26 is connected to the connecting tube 21 of the connector 11. When the connector 11 of the endoscope 2 is connected to the liquid supply device body 1, the air supply passage 20 of the connector 11 is connected to the air supply pump 4. When the cap 29 is to be connected to the connecting member 21, the second annular member 23 is initially engaged with the cap 33. When in this state the cap 30 is further pushed in, a stepped section of the connecting portion 40 of the cap 33 abuts against the end face of the second annular member 23 so that the cap 33 is held by the second annular member 23 in the engaged position. With the cap 30 is further pushed in, the piston section 41 within the cylinder section 32 is moved against the compression coil spring 43 away from the bottom 30a of the cap 30. That is, the air supply cap 30 is moved forward relative to the liquid supply cap 33. When the cap 30 is moved to a normal insertion position within the first annular member 22, the C-ring 36 is latched by a latching portion 22d which is formed by an annular rib projected from the forward inner end portion of the annular member 22 so that the cap 30 is connected to the annular member 22. As a result, the interior of the cylinder section 32 on the side of the bottom 30a communicates with the interior of the cylinder section 32 on the side of the air supply tube 27 through the communication hole 42, and the air supply tube 27 communicates with the air supply passage 20 of the connector 11 through the interior of the cylinder section 32, the gap between the opening 31 of the cap 30 and the cap 33, the communication hole 39 of the projection 38 and the interior of the first annular member 22. The liquid supply tube 28 communicates with the liquid supply passage 16 of the connector 11 through the cap 33 and the interior of the second annular member 23. As compressed air sent from the air supply pump 4 is introduced through the air supply passage 20 of the connector 11 and the air supply tube 27 of the connecting tube 26 into the sealed container 25 of the liquid supply tank 5, the internal pressure of the sealed container 25 is increased to permit the liquid in the sealed container 25 to be supplied into the liquid supply passage 16 of the endoscope 2.

When the cap 29 of the connecting tube 26 is removed from the connector 11 of the endoscope 2 after completion of a liquid supply operation, the liquid supply cap 33 is pushed forward by a spring force of the compression coil spring 43, causing the piston section 41 within the cylinder of the cap 30 to be urged against the O-ring 35 on the bottom 30a of the cap 30 as shown in Fig. 3, thus shutting off communication between the opening 31 in the bottom 30a and the communication holes 42 by the O-ring 35 and piston section 41. Since an air supply from the air supply tube 27 into the sealed container 25 is stopped, the internal pressure of the sealed container 25 is not increased. Consequently, the liquid in the sealed container 25 is not supplied into the liquid supply tube 28, thus preventing the liquid within the tank 5 from flowing out through the liquid supply tube 28 on the basis of a "siphon" principle as uid supply device. By merely removing the cap 29 for the connecting tube 26 away from the connecting member 21 for the connector 11, a liquid leakage from the cap 33 can be ted, thus assuring an easy, error-free operation.

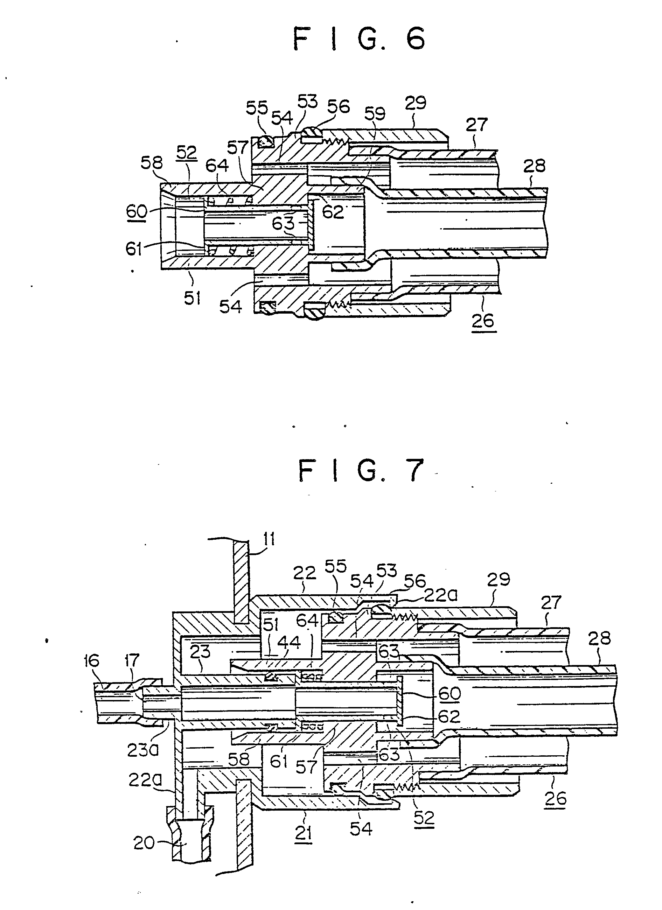

Another embodiment of this invention will be explained by referring to Figs. 6 and 7.

A closable valve mechanism 52 is disposed within a liquid supply cap 51 and the opening and closing of a liquid supply tube 28 are achieved by the valve mechanism 52. The liquid supply cap 51 is formed integral with, and coaxial with, a large-diameter air supply cap 53. A plurality of communication holes 54 are provided in the end wall of the cap 53 along the axial direction of the cap 53 to permit communication between the interior of the cap 53 and the outside. An O- and C-ring (55 and 56) are fitted in the recessed portions of the outer periphery of the cap 53. The liquid supply cap 51 is comprised of a large-diameter section 57 located in the intermediate position and acting as an end wall of the cap 53, a small-diameter, first connecting section 58 extending outwardly from the end wall of the cap 53, and a small-diameter, second connecting section 59 extending in the cap 53. A communication hole defined by the large-diameter section 57 is smaller in diameter than the communication holes defined by the connecting sections 58 and 59. A cylindrical movable member 60 with a bottom is air-tightly inserted into the large-diameter section 57 of the cap 51 and adapted to be moved back and forth along the axial direction of the cap 53. The movable member 60 has a flange-equipped piston section 61 located in the open end portion of the first connecting portion 58 and a large-diameter latching portion 62 adapted to be engaged with the bottom of the second connecting section 59. A plurality of communication holes 63 are formed in that portion of the peripheral wall of the movable member 60 which is located close to the latching section 62. Within the first connecting section 58 of the cap 51 a compression coil spring 64 is located between the piston section 61 and the end of the large-diameter section 57. In the nonuse state in which the first connecting section 58 of the cap 51 is not engaged with the second annular member 23 of the connector 11, the movable member 60 is urged by the compression coil spring 64 to the left in Fig. 6, causing the latching member 62 to abut against the corresponding end of the large-diameter section 57 of the liquid supply cap 51. In this state, the movable member is held such that the respective communication holes 63 are blocked by the inner surface of the large-diameter section 57. When the connecting portion 58 of the liquid supply cap 51 is engaged with the second annular member 23 of the connector 11, the movable member 60 is pushed to the right by the second annular member 23, as shown in Fig. 7, against an urging force of the compression coil spring 64, causing the communication holes 63 of the movable member to be exposed on the side of the second connecting section 59 to permit communication to be effected between the liquid supply tube 28 and the second annular member 23 through the movable member 60.

When a cap 29 of a connecting tube 26 is connected to the connecting member 21 of the connector 11 as shown in Fig. 7, the air supply passage 20 of the connector 11 communicates with the air supply tube 27, causing the interior of the air supply tank 25 to be compressed to send out the liquid in the liquid supply tank 5 through the liquid supply tube 28. As in this state the movable member 60 is pushed in by the second annular member 23 to expose the communication holes on the side of the second connecting section 59, the liquid sent through the liquid supply tube 28 can be supplied into the liquid supply passage 16 of the endoscope 2 through the communication holes 63 of the movable member 60. When the cap 29 for the connecting tube 26 is removed from the connecting member 21 of the connector 11, the movable member 60 is moved by the compression coil spring 64 back to the original position, blocking up the communication holes 63 of the movable member 60 by the inner surface of the large-diameter section 57 and thus immediately stopping a liquid supply from the liquid supply cap 51. When the cap 29 for the connecting tube 26 is detached from the connector 11 of the endoscope 2, the liquid cap 51 can be directly blocked up. Even in this case, the liquid in the liquid supply tank 5 can be positively prevented from flowing out through the liquid supply tube 28 because of the siphon principle.

Although this invention has been explained in connection with the above-mentioned embodiments, it is not restricted to the above-mentioned embodiments. For example, an arrangement for connecting the cap 29 for the connecting tube 26 to the connecting member 21 for the connector 11 may be properly modified. By removing the cap 29 for the connecting tube 26 from the connecting member 21 of the connector 11 the air supply tube 27 and liquid supply tube 28 may be blocked. This invention can be reduced to practice without departing from the scope of this invention, i.e. when the liquid supply tube is removed from the endoscope, the liquid within the liquid supply tank can be prevented from flowing out through the liquid supply tube on the basis of the siphon principle.

**Claims**

1. A liquid supply device for an endoscope which includes a connecting member (21) having a liquid supply passage (16) connected to a liquid supply passage in the endoscope and an air supply passage (20), comprising:

a closed liquid supply tank (5) for holding a liquid therein;

connecting means (30) detachably connectable to said connecting member (21);

a liquid supply tube (28) connected at one end of the connecting means (30) and located at the other end in the liquid in the liquid supply tank (5);

air supply means (4) connected to the air supply passage; and

an air supply tube (27) connectable at one end to the air supply means through the connecting means and the air supply passage in the connecting member and located at the other end in air in the liquid supply tank for causing pressure in the liquid supply tank to be increased by air from the air supply means to permit the liquid within the tank to be sent into the liquid supply tube and thence to the endoscope via the liquid supply device,

the device further including a valve mechanism (34) including a first tube (23) on the connecting member (21) and communicating with the liquid supply passage (16), and a second tube (40) on the connecting means (30) and communicating with the liquid supply tube (28), the second tube (40) including and an enlarged bore portion and a flange (41) which is spring biased against a sealing ring (35) and has an opening (42) for the passage of air, the opening being closed in the normal position of the flange in which the air supply tube is closed to the atmosphere and disconnected from the air supply passage, the valve mechanism being adapted, when the connecting means (30) is connected to the connecting member (21) to sealingly engage the first tube (23) within the enlarged bore portion of the second tube (40) and thereby connect the liquid supply tube (28) to the liquid supply passage (16) and to bias the flange away from the sealing ring to open the normally closed opening and thereby connect the air supply tube (27) to the air supply passage (20) and adapted, when the connecting means (30) is removed from the connecting member (21), to automatically shut off communication between the air supply tube·(27) and the air supply passage (20) by re-closing the opening (42) and, by disconnecting the first and second tubes to break the connection between the liquid supply tube (28) and the liquid supply passage (16) after shutting off communication between the air supply tube (27) and the atmosphere, so that the liquid supply tube (28) may communicate with the atmosphere.

2. A liquid supply device according to claim 1, in which said valve mechanism (34) comprises:

an air supply cap (30) connected to the other end of the air supply tube and connectable to the air supply passage;

a liquid supply cap (33) connected to the other end of the liquid supply tube, connectable to the liquid supply passage and movable relative to the air supply cap;

urging means (43) for urging the liquid supply cap in one direction toward the air supply cap;

closable means for permitting communication to be effected between the liquid supply tube and the outside when the liquid supply cap is moved by the urging means in said one direction, the liquid supply tube connected to the air supply passage through connection to the outside.

3. A liquid supply device according to claim 2, in which said liquid supply cap (33) is, after being connected to the connecting section of the endoscope, moved in the other direction against an urging force of said urging means.

4. A liquid supply device according to claim 3, in which said liquid supply tube (28) is provided in the air supply tube (27) and said liquid supply cap is provided in the air supply cap (30) such that it is coaxial with the liquid supply tube.

5. A liquid supply device for an endoscope which includes a connecting member (21) having a liquid supply passage (16) connected to a liquid supply passage in the endoscope, and an air supply passage (20), said liquid supply device comprising:

a closed liquid supply tank (5) for holding a liquid therein;

connecting means (53) detachably connectable to said connecting member (21);

a liquid supply tube (28) connected at one end to the connecting means (53) and located at the other end in liquid in the liquid supply tank;

air supply means (4) connected to said air supply passage;

an air supply tube (27) connectable at one end to the air supplying means through the connecting means and the air supply passage in the connecting member and located at the other end in air in the liquid supply tank for causing pressure in the liquid supply tank to be increased by air from the air supply means to permit the liquid within the tank to be sent to the liquid supply tube and thence to the endoscope via the liquid supply device; and

a valve mechanism including:

a first tube (23) on the connecting member (21) and communicating with the liquid supply passage (16), and, on the connecting means (53), first and second annular members (58, 59) separated by a thick wall (57), a second tube (60) sealing slidable through the wall and having a flange (61) at one end thereof and within first annular member (58) and spring-biased away from the wall, the second tube being closed by an end wall (62) at the other end thereof and within the second annular member (59) and having side openings (63), wherein, in the disengaged condition of the connecting member and the connecting means the openings (63) are closed by the thick wall, and, in the engaged condition thereof, the first tube sealingly engages within the first annular member and pushes the second tube against the spring bias such that the openings (63) enter the space within the second annular member, which communicates with the liquid supply tube, to enable a liquid passage to be established between the liquid supply passage and the liquid supply tube, the engaged condition also establishing communication between the air supply passage and the air supply tube.

6. A liquid supply device according to claim 5, in which said valve mechanism (34) comprises:

an air supply cap connected to the other end of the air supply tube and connectable to the air supply passage;

a liquid supply cap (51) connected to the other end of the liquid supply passage, connectable to the liquid passage and fixed on the air supply cap;

communication means (54) provided in the air supply cap for causing the air supply tube to normally communicate with the outside; and means (60) selectively openable and closable to permit and shut off communication between the liquid supply cap (51) and the liquid supply tube (28), whereby when the connecting means is connected to the connecting section of the endoscope the openable and closable means permits communication to be effected between the liquid supply tube and the liquid supply passage through the liquid supply cap.

7. A liquid supply device according to claims 6, in which said openable and closable means has a piston section disposed in the liquid supply cap and movable along the liquid supply cap to permit a selective opening and closing to be effected between the liquid supply cap and the liquid supply tube.

## Revendications

1. Dispositif d'alimentation en liquide pour endoscope incluant un organe de connexion (21) ayant un passage d'alimentation en liquide (16) connecté à un passage d'alimentation en liquide situé dans l'endoscope et un passage d'alimentation en air (20) comprenant:

un réservoir d'alimentation en liquide (5) fermé pour contenir un liquide;

un dispositif de connexion (30) pouvant être connecté de façon amovible audit organe de connexion (21);

un tube d'alimentation en liquide (28) dont l'une extrémité est connectée au dispositif de connexion (30) et dont l'autre extrémité est située dans le liquide se trouvant dans le réservoir d'alimentation en liquide (5);

un dispositif d'alimentation en air (4) connecté au passage d'alimentation en air; et

un tube d'alimentation en air (27) pouvant être connecté à une extrémité au dispositif d'alimentation en air au moyen du dispositif de connexion et du passage d'alimentation en air dans l'organe de connexion et dont l'autre extrémité est située dans l'air se trouvant dans le réservoir d'alimentation en liquide afin d'accroître la pression dans le réservoir d'alimentation en liquide par l'air du dispositif d'alimentation en air pour permettre au liquide contenu dans le réservoir d'être envoyé dans le tube d'alimentation en lqiuide puis dans l'endoscope via le dispositif d'alimentation en liquide,

en outre, le dispositif comprenant un mécanisme de vanne (34) incluant un premier tube (23) situé sur l'organe de connexion (21) et communiquant avec le passage d'alimentation en liquide (16) et un second tube (40) situé sur le dispositif de connexion (30) et communiquant avec le tube d'alimentation en liquide (28), le second tube (40) incluant à la fois une section de perçage agrandie et un collet (41) précontraint au moyen d'un ressort contre une rondelle d'étanchéité (35) et a une ouverture (42) pour le passage de l'air, l'ouverture étant fermée dans la position normale du collet dans laquelle le tube d'alimentation en air est fermé à l'atmosphère et disconnecté du passage d'alimentation en air, le mécanisme de vanne étant adapté, lorsque le dispositif de connexion (30) est connecté à l'organe de connexion (21) pour mettre en prise de façon étanche le premier tube (23) à l'intérieur de la section de perçage agrandie du second tube (40) et ainsi connecter le tube d'alimentation en liquide (28) au passage d'alimentation en liquide (16) et pour écarter le collet de la rondelle d'étanchéité pour ouvrir l'ouverture normalement fermée et ainsi connecter le tube d'alimentation en air (27) au passage d'alimentation en air (20) et adapté, lorsque le dispositif de connexion (30) est enlevé de l'organe de connexion (21), à la communication automatiquement fermée entre le tube d'alimentation en air (27) et le passage d'alimentation en air (20) en refermant l'ouverture (42) et en déconnectant le premier et le second tube pour interrompre la connexion entre le tube d'alimentation en liquide (28) et le passage d'alimentation en liquide (16) après avoir fermé la communication entre le tube d'alimentation en air (27) et l'atmosphère, de façon à ce que le tube d'alimentation en liquide (28) puisse communiquer avec l'atmosphère.

2. Dispositif d'alimentation en liquide selon la revendication 1, dans lequel ledit mécanisme de vanne (34) comprend:

un capuchon d'alimentation en air (30) connecté à l'autre extrémité du tube d'alimentation en air et pouvant être connecté au passage d'alimentation en air;

un capuchon d'alimentation en liquide (33) connecté à l'autre extrémité du tube d'alimentation en liquide, pouvant être connecté au passage d'alimentation en liquide et amovible par rapport au capuchon d'alimentation en air;

un dispositif tendeur (43) pour pousser le capuchon d'alimentation en liquide dans une direction vers le capuchon d'alimentation en air;

un dispositif de fermeture pour permettre de créer une communication entre le tube d'alimentation en liquide et l'extérieur lorsque le capuchon d'alimentation en liquide est poussé par le dispositif tendeur dans ladite direction, le tube d'alimentation en liquide étant connecté au passage d'alimentation en air à travers la connexion vers l'extérieur.

3. Dispositif d'alimentation en liquide selon la revendication 2, dans lequel ledit capuchon d'alimentation en liquide (33) est, après avoir été connecté à la section de connexion de l'endoscope, poussé dans l'autre direction contre une force de pression dudit dispositif tendeur.

4. Dispositif d'alimentation en liquide selon la revendication 3, dans lequel ledit tube d'alimentation en liquide (28) est fourni dans le tube d'alimentation en air (27) et ledit capuchon d'alimentation en liquide est fourni dans le capuchon d'alimentation en air (30) de façon à être coaxial par rapport au tube d'alimentation en liquide.

5. Dispositif d'alimentation en liquide pour endoscope incluant un organe de connexion (21) ayant un passage d'alimentation en liquide (16) connecté à un passage d'alimentation en liquide situé dans l'endoscope, et un passage d'alimentation en air (20), ledit dispositif d'alimentation en liquide comprenant:

un réservoir d'alimentation en liquide (5) fermé pour contenir un liquide;

un dispositif de connexion (53) pouvant être connecté de façon amovible audit organe de connexion (21),

un tube d'alimentation en liquide (28) dont l'une extrémité est connectée au dispositif de connexion (53) et dont l'autre extrémité est située dans le liquide se trouvant dans le réservoir d'alimentation en liquide;

un dispositif d'alimentation en air (4) connecté audit passage d'alimentation en air;

un tube d'alimentation en air (27) pouvant être connecté à une extrémité au dispositif d'alimentation en air au moyen du dispositif de connexion et du passage d'alimentation en air dans l'organe de connexion et dont l'autre extrémité est située dans l'air se trouvant dans le réservoir d'alimentation en liquide afin d'accoître la pression dans le réservoir d'alimentation en liquide par l'air du dispositif d'alimentation en air pour permettre au liquide contenu dans le réservoir d'être envoyé dans le tube d'alimentation en liquide puis dans l'endoscope via le dispositif d'alimentation en liquide; et

un mécanisme de vanne incluant:

un premier tube (23) situé sur l'organe de connexion (21) et communiquant avec le passage d'alimentation en liquide (16), et, sur le dispositif de connexion (53), un premier et un second organe annulaire (58; 59) séparés par une paroi épaisse (57), un second tube (60) étanche pouvant être glissé de façon étanche à travers la paroi et ayant un collet (61) à une extrémité et à l'intérieur du premier organe annulaire (58) et étant écarté de la paroi sous précontrainte, le second tube étant fermé par une paroi finale (62) à l'autre extrémité et à l'intérieur du second organe annulaire (59) et ayant des ouvertures latérales (63), les ouvertures (63) étant fermées par une paroi épaisse lorsque l'organe de connexion et le dispositif de connexion sont désengrénés et, lorsqu'ils sont engrénés, le premier tube étant mis en prise hermétiquement à l'intérieur du premier organe annulaire et poussant le second tube contre la précontrainte de façon à ce que les ouvertures (63) entrent dans l'espace à l'intérieur du second organe annulaire communiquant avec le tube d'alimentation en liquide pour permettre une création de passage de liquide entre le passage d'alimentation en liquide et le tube d'alimentation en liquide, la position engrénée permettant d'établir une communication entre le passage d'alimentation en air et le tube d'alimentation en air.

6. Dispositif d'alimentation en liquide selon la revendication 5, dans lequel ledit mécanisme de vanne (34) comprend:

un capuchon d'alimentation en air connecté à l'autre extrémité du tube d'alimentation en air et pouvant être connecté au passage d'alimentation en air;

un capuchon d'alimentation en liquide (51) connecté à l'autre extrémité du passage d'alimentation en liquide, pouvant être connecté au passage d'alimentation en liquide et fixé sur le capuchon d'alimentation en air;

un dispositif de communication (54) fourni dans le capuchon d'alimentation en air pour permettre au tube d'alimentation en air de communiquer normalement avec l'extérieur; et

un dispositif (60) pouvant être ouvert et fermé de façon selective pour permettre et interrompre la communication entre le capuchon d'alimentation en liquide (51) et le tube d'alimentation en liquide (28), le dispositif d'ouverture et de fermeture permettant à la communication de s'effectuer entre le tube d'alimentation en liquide et le passage d'alimentation en liquide à travers le capuchon d'alimentation en liquide quand le dispositif de connexion est connecté à la section de connexion de l'endoscope.

7. Dispositif d'alimentation en liquide selon la revendication 6, dans lequel ledit dispositif d'ouverture et de fermeture a une section à piston disposée dans le capuchon d'alimentation en liquide et amovible le long du capuchon d'alimentation en liquide pour permettre à une ouverture et fermeture sélective de s'effectuer entre le capuchon d'alimentation en liquide et le tube d'alimentation en liquide.

**Patentansprüche**

1. Flüssigkeitszuführungsvorrichtung für ein Endoskop, welches ein Verbindungsteil (21) umfaßt, das einen mit einem Flüssigkeitszuführungskanal in dem Endoskop verbundenen Flüssigkeitszuführungskanal (16) und einen Luftzuführungskanal (20) aufweist, mit:

einem geschlossenen Flüssigkeitszuführungs-Tank (5) zur Aufbewahrung einer Flüssigkeit;

einer Verbindungsvorrichtung (30), welche mit dem Verbindungsteil (21) abnehmbar verbindbar ist;

einem Flüssigkeitszuführungsrohr (28) dessen eines Ende mit der Verbindungsvorrichtung (30) verbunden und dessen anderes Ende in der Flüssigkeit im Flüssigkeitszuführungs-Tank (5) angeordnet ist;

einer Luftzuführungsvorrichtung (4), welche mit dem Luftzuführungskanal verbunden ist; und

einem Luftzuführungsrohr (27), welches an einem Ende mit der Luftzuführungsvorrichtung durch die Verbindungsvorrichtung und dem Luftzuführungskanal in dem Verbindungsteil verbindbar ist und dessen anderes Ende in der Luft in dem Flüssigkeitszuführungs-Tank angeordnet ist zur Anhebung des Drucks im Flüssigkeitszuführungs-Tank mittels Luft von der Luftzuführungsvorrichtung, um zu ermöglichen, daß die Flüssigkeit im Tank in das Flüssigkeitszuführungsrohr gefördert wird und von dort dem Endoskop über die Flüssigkeitszuführungsvorrichtung zugeführt wird, wobei die Vorrichtung weiterhin einen Absperrmechanismus (34) aufweist, der ein erstes Rohr (23) an dem Verbindungsteil (21) aufweist und mit dem Flüssigkeitszuführungskanal (16) in Verbindung steht, und ein zweites Rohr (40) an der Verbindungsvorrichtung aufweist und mit dem Flüssigkeitszuführungsrohr (28) in Verbindung steht, wobei das zweite Rohr (40) sowohl einen vergrößerten Bohrungsbereich, als auch einen Flansch (41) aufweist, der mittels Feder gegen einen Dichtring (35) vorgespannt ist und für das Hindurchströmen von Luft eine Öffnung (42) aufweist, wobei die Öffnung in der Normalstellung des Flansches geschlossen ist, in der das Luftzuführungsrohr gegenüber der Umgebung geschlossen und die Verbindung zum Luftzuführungskanal unterbrochen ist, wobei der Absperrmechanismus derart ausgebildet ist, daß er, wenn die Verbindungsvorrichtung (30) mit dem Verbindungsteil (21) verbunden ist, dichtend in Eingriff mit einem ersten Rohr (23) innerhalb des vergrößerten Bohrungsbereiches des zweiten Rohres (40) steht und dabei das Flüssigkeitszuführungsrohr (28) mit dem Flüssigkeitszuführungskanal (16) verbindet und den Flansch von dem Dichtring wegdrückt, um die normalerweise geschlossene Öffnung zu öffnen und dabei das Luftzuführungsrohr (27) mit dem Luftzuführungskanal (20) zu verbinden und weiterhin derart ausgebildet ist, daß, wenn die Verbindungsvorrichtung (30) von dem Verbindungsteil (21) wegbewegt ist, die Verbindung zwischen dem Luftzuführungsrohr (27) und dem Luftzuführungskanal (20) durch erneutes Schließen der Öffnung (42) automatisch unterbrochen wird, und daß durch Trennen des ersten und zweiten Rohres die Verbindung zwischen dem Flüssigkeitszuführungsrohr (28) und dem Flüssigkeitszuführungskanal (16) nach Unterbrechen der Verbindung zwischen dem Luftzuführungsrohr (27) und der Umgebung unterbrochen wird, so daß das Flüssigkeitszuführungsrohr (28) in Verbindung mit der Umgebung treten kann.

2. Flüssigkeitszuführungsvorrichtung nach Anspruch 1, wobei der Absperrmechanismus (34) folgendes aufweist:

eine Luftzuführungskappe (30), welche mit dem anderen Ende des Luftzuführungsrohr verbunden und mit dem Luftzuführungskanal verbindbar ist;

eine Flüssigkeitszuführungskappe (33), die mit dem anderen Ende des Flüssigkeitszuführungsrohr verbunden ist, mit dem Flüssigkeitszuführungskanal verbindbar und relativ zu der Luftzuführungskappe bewegbar ist;

eine Spannvorrichtung (43) zum Drängen der Flüssigkeitszuführungskappe in eine Richtung gegen die Luftzuführungskappe;

eine schließbare Vorrichtung zur Ermöglichung einer zwischen dem Flüssigkeitszuführungsrohr und der Aussenseite wirkenden Verbindung, wenn die Flüssigkeitszuführungskappe durch die Spannvorrichtung in diese eine Richtung bewegt wird, wobei das Flüssigkeitszuführungsrohr durch Verbindung zur Außenseite mit dem Luftzuführungskanal verbunden ist.

3. Flüssigkeitszuführungsvorrichtung nach Anspruch 2, wobei die Flüssigkeitszuführungskappe (33) nachdem sie mit dem Verbindungsbereich des Endoskops verbunden worden ist, in die andere Richtung gegen eine Druckkraft der Spannvorrichtung bewegt wird.

4. Flüssigkeitszuführungsvorrichtung nach Anspruch 3, wobei das Flüssigkeitszuführungsrohr (28) in dem Luftzuführungsrohr (27) angeordnet ist und die Flüssigkeitszuführungskappe in der Luftzuführungskappe (30) derart angeordnet ist, daß sie sich koaxial zum Flüssigkeitszuführungsrohr erstreckt.

5. Flüssigkeitszuführungsvorrichtung für ein Endoskop, welches ein Verbindungsteil (21) umfaßt, das einen mit einem Flüssigkeitszuführungskanal in dem Endoskop verbundenen Flüssigkeitszuführungskanal (16) und einen Luftzuführungskanal (20) aufweist, wobei die Flüssigkeitszuführungsvorrichtung folgendes aufweist:

einen geschlossenen Flüssigkeitszuführungs-Tank (5) zur Aufbewahrung einer Flüssigkeit;

eine Verbindungsvorrichtung (53), welche mit dem Verbindungsteil (21) abnehmbar verbindbar ist;

ein Flüssigkeitszuführungsrohr (28) dessen eines Ende mit der Verbindungsvorrichtung (26) verbunden und dessen anderes Ende in der Flüssigkeit im Flüssigkeitszuführungs-Tank angeordnet ist;

eine Luftzuführungsvorrichtung (4), welche mit dem Luftzuführungskanal verbunden ist;

ein Luftzuführungsrohr (27), welches an einem Ende mit der Luftzuführungsvorrichtung durch die Verbindungsvorrichtung und dem Luftzuführungskanal in dem Verbindungsteil verbindbar ist und dessen anderes Ende in der Luft in dem Flüssigkeitszuführungs-Tank angeordnet ist zur Anhebung des Drucks im Flüssigkeitszuführungs-Tank mittels Luft von der Luftzuführungsvorrichtung, um zu edrmöglichen, daß die Flüssigkeit im Tank in das Flüssigkeitszuführungsrohr gefördert wird und von dort dem Endoskop über die Flüssigkeitszuführungsvorrichtung zugeführt wird; und

einen Absperrmechanismus, der folgendes umfaßt:

ein erstes Rohr (23) an dem Verbindungsteil (21), das mit dem Flüssigkeitszuführungskanal (16) in Verbindung steht und an der Verbindungsvorrichtung ein erstes und ein zweites Ringteil (58, 59), die durch eine dicke Wand (57) voneinander getrennt sind, ein zweites Rohr (60), das sich dichtend gleitbewegbar durch die Wand erstreckt und an einem Ende innerhalb des ersten Ringteiles (58) einen Flansch (61) aufweist, und unter Vorspannung weg von der Wand steht, wobei das zweite Rohr von einer Abschlußwand (62) an dem anderen Ende und innerhalb des zweiten Ringteiles (59) geschlossen ist, und Seitenöffnungen (63) aufweist, wobei die Seitenöffnungen (63) in der Außer-Eingriff-Stellung des Verbindungsteiles und der Verbindungsvorrichtung durch die dicke Wand geschlossen sind, und in der Eingriffsstellung das erste Rohr dichtend in Eingriff innerhalb des ersten Ringteiles steht und das zweite Rohr gegen die vorgespannte Feder derart drückt, daß die Öffnungen (63) in dem Raum innerhalb des zweiten Ringteiles eintreten, der mit dem Flüssigkeitszuführungsrohr in Verbindung steht, um die Schaffung eines Flüssigkeitskanals zwischen dem Flüssigkeitszuführungskanal und dem Flüssigkeitszuführungsrohr zu ermöglichen, wobei die Eingriffsstellung auch eine Verbindung zwischen dem Luftzuführungskanal und dem Luftzuführungsrohr schafft.

6. Flüssigkeitszuführungsvorrichtung nach Anspruch 5, wobei der Absperrmechanismus (34) folgendes aufweist:

eine Luftzuführungskappe, die mit dem anderen Ende des Luftzuführungsrohr verbunden und mit dem Luftzuführungskanal verbindbar ist;

eine Flüssigkeitszuführungskappe (51), die mit dem anderen Ende des Flüssigkeitszuführungskanal verbunden ist, mit dem Flüssigkeitskanal verbindbar und auf der Luftzuführungskappe befestigt ist;

eine Verbindungsvorrichtung (54), welche in der Luftzuführungskappe angeordnet ist, um das Luftzuführungsrohr zu verursachen normalerweise in Verbindung mit der Umgebung zu stehen; und

eine Vorrichtung (60), die wahlweise öffenbar und schließbar ist, um eine Verbindung zwischen der Flüssigkeitszuführungskappe (51) und dem Flüssigkeitszuführungsrohr (28) zu erlauben und zu unterbrechen, wodurch, wenn die Verbindungsvorrichtung mit dem Verbindungsbereich des Endoskops verbunden ist, die Öffnungs- und Schließvorrichtung eine zwischen dem Flüssigkeitszuführungsrohr und dem Flüssigkeitszuführungskanal durch die Flüssigkeitszuführungskappe hindurch wirksame Verbindung erlaubt.

7. Flüssigkeitszuführungsvorrichtung nach Anspruch 6, wobei die Öffnungs- und Schließvorrichtung einen Kolbenbereich aufweist, der in der Flüssigkeitszuführungskappe angeordnet und entlang der Flüssigkeitszuführungskappe bewegbar ist, um eine wahlweise Öffnung und Schließung, welche zwischen der Flüssigkeitszuführungskappe und dem Flüssigkeitszuführungsrohr bewirkt wird, zu erlauben.

# F I G. 1

# F I G. 2

FIG. 3

FIG. 4

FIG. 5

2

# F I G. 6

# F I G. 7